Europäisches Patentamt

European Patent Office

Office européen des brevets

(19)

(11) Publication number: **0 272 878**
**A2**

# EUROPEAN PATENT APPLICATION

(12)

(21) Application number: **87311094.4**

(22) Date of filing: **16.12.87**

(51) Int. Cl.⁴: **A 61 K 7/16**

(30) Priority: **16.12.86 JP 300759/86**

(43) Date of publication of application:
**29.06.88 Bulletin 88/26**

(84) Designated Contracting States:
**BE CH DE FR GB IT LI NL SE**

(71) Applicant: **SUNSTAR KABUSHIKI KAISHA**
**3-1, Asahi-machi**
**Takatsuki-shi Osaka-fu (JP)**

(72) Inventor: **Kobayashi, Yuji**
**No. 10-511, Soujijidai Ibaraki-shi**
**Osaka-fu (JP)**

(74) Representative: **Ford, Michael Frederick et al**
**MEWBURN ELLIS & CO. 2/3 Cursitor Street**
**London EC4A 1BQ (GB)**

(54) Dentifrice composition.

(57) A dentifrice composition to be packed in a container when the composition is liable to lose its flavor which comprises as a flavor retaining ingredient a triglyceride of straight or branched chain fatty acids having 6 to 12 carbon atoms, respectively, and other conventional dentifrice ingredients.

EP 0 272 878 A2

**Description**

DENTIFRICE COMPOSITION

The present invention relates to a dentifrice composition. More particularly, the present invention concerns retention of flavour even if the composition is packed in a container wherein the composition is liable to lose its flavour, and maintaining improved feeling by the user i.e. sensory perception when the composition is used.

BACKGROUND OF THE INVENTION

In a dentifrice composition, flavor is formulated to mask unpleasant taste or odor derived from other ingredients contained in the composition and, upon using, to provide pleasant feeling and improved feeling for use thereof. Therefore, it can be said that flavor is one of essential ingredients of a dentifrice composition.

Generally, flavor is volatilized in the nostrils and the mouth and is perceived by the sense of smell and taste. Therefore, almost all flavors are composed of volatile substances having low boiling points. Accordingly, flavor formulated in a dentifrice composition is in a volatilizable state and, if it is volatilized before use, the effect of formulation of flavor is lost and improvement of feeling for use can not be expected.

On the other hand, dentifrice compositions are marketed by packing in various containers. Aluminum tubes which have been generally used heretofore are impermeable to volatile components of flavor and scarcely involve problems such as spoilage of feeling for use caused by volatilization of flavor. However, recently, in view of convenience and the like, plastic containers such as laminate tubes, plastic tubes, plastic pump dispenser-containers have been used and, because they are permeable to volatile components of flavor, there is a problem that flavor is volatilized to spoil feeling for used, which results in loss of commercial value during storage for a long period of time.

In order to solve such a problem, addition of various flavor retaining ingredients to a dentifrice composition has been studied, but no one is found to be sufficient.

For example, flavor components having high boiling point (e.g. natural flavor such as musk, oakmos or orris, and synthetic flavor such as coumarin, vanillin) are proposed to be used with conventional flavor for dentifrice. However, because they possess their own characteristic oder, they can not be used in an effective amount for flavor retention. Further, there is a problem that it is difficult to admix them with conventional flavor for dentifrice such as peppermint type and spearmint type flavor.

Although polyhydric alcohols such as glycerin or propylene glycol are used as flavor retention ingredients, they have such characteristic taste and, when they are used in an effective amount for flavor retention, they are liable to spoil feeling for use. In addition, although plasticizers such as phthalate ester are suggested to be used, they have a problem on safety.

Recently, there have been proposed to use a higher fatty acid alcohol having 16 to 28 carbon atoms which is liquid at room temperature (Japanese Patent Laid Open Publication No. 61-151114) or a premix prepared by admixing polyvinyl pyrrolidone in a flavor oil (Japanese Patent Laid Open Publication No. 61-243012). However, their effects are unclear.

Under these circumstances, the present inventor has studied flavour retention ingredients in order to solve problem due to volatilization of flavour contained in a dentifrice composition. As the result, it has been found that a certain triglyceride is suitable as a flavour retention component.

The present invention aims to provide a dentifrice composition for which loss of flavour is prevented or reduced, so that even if it is packed in a container wherein the composition is liable to lose its flavour, it can maintain improved feeling for use, i.e. it can maintain properties which are beneficial to the user's sensory perception of the product.

SUMMARY OF THE INVENTION

According to the present invention, there is provided a dentifrice composition suitable to be packed in a container wherein the composition is liable to lose its flavour, which comprises as a flavour retaining ingredient a triglyceride of straight or branched chain fatty acids having 6 to 12 carbon atoms, respectively, and other conventional dentifrice ingredients.

By adding a triglyceride of fatty acids having 6 to 12 carbon atoms as a flavour retaining ingredient, the dentifrice composition of the present invention can be prevented from loss of flavour and, even if it is packed in a container wherein the composition is liable to lose its flavour, it can maintain improved feeling for use for a long period of time. Further, the composition of the present invention does not have such characteristic odor as that encountered with conventional flavour retention ingredients.

BRIEF EXPLANATION OF DRAWING

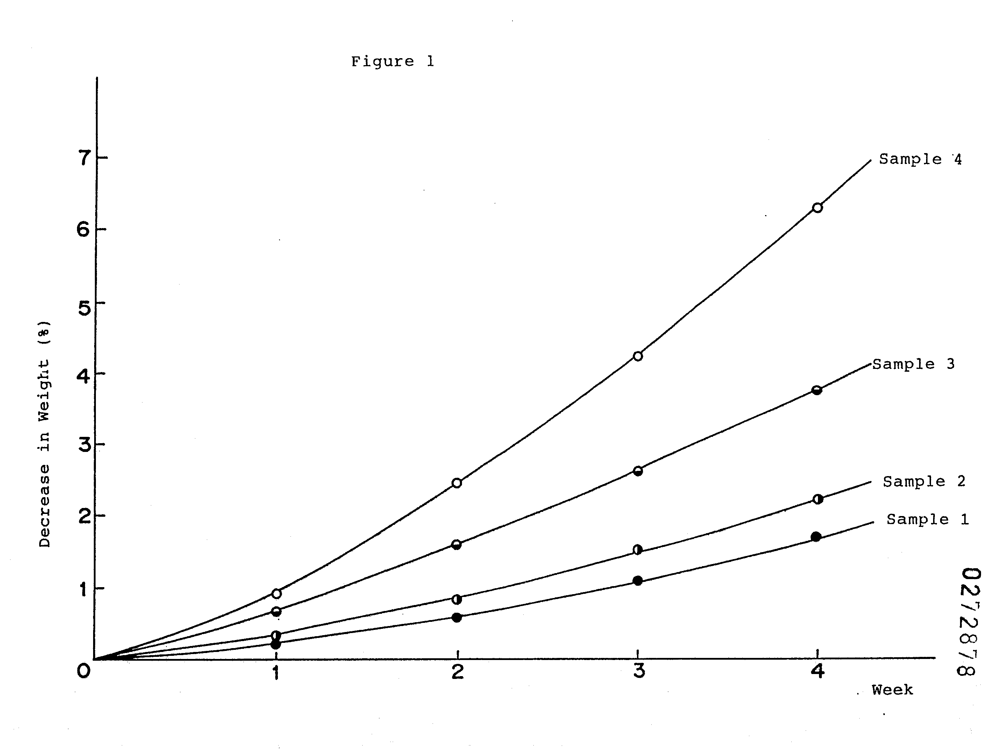

Figure 1 is a graph illustrating the result of a permeation test of dentifrice flavour from a polyethylene tube with time as described hereinafter.

DETAILED DESCRIPTION

The composition of this invention is suitable for packing in a container wherein the composition is liable to lose its flavour for example, plastic containers such as laminate tubes, plastic tubes, plastic pump dispenser-containers.

The triglyceride used as the flavor retention ingredient in the present invention is mainly composed of straight or branched fatty acids having 6 to 12 carbon atoms, respectively. Examples of such straight chain fatty acid include caproic acid, caprylic acid, pelargonic acid, undecanoic acid, lauric acid and the like, and examples of the branched chain fatty acid include iso acids and anti-iso acids of the straight-chain fatty acids. When number of carbon atoms contained in the fatty acid is less than 6, a triglyceride with high purity can not be obtained and it is not suitable to be used for a dentifrice because of its characteristic odor. When the number of carbon atoms is more than 12, it is not preferred in view of stability of a resulting dentifrice because it sometimes causes rough texture, separation and the like. The positions of these fatty acids in the triglyceride are not specifically limited. That is, the fatty acids at $\alpha$-and $\beta$-positions may be the same or different, or triglycerides with different fatty acid compositions can be optionally combined. Particularly, in the present invention, a triglyceride of saturated fatty acids having 8 and/or 10 carbon atoms, more particularly, a triglyceride of a saturated fatty acid having 8 carbon atoms and that having 10 carbon atoms in which the weight ratio thereof is 10 : 1 to 1 : 8 is preferred because of its excellent flavor retention effect. The triglyceride used in the present invention is commercially available. For example, Sunfat MCT-6, Sunfat GTC, Sunfat 10D, Onoil GTE-D (manufactured by Taiyo Kagaku Co., Ltd., Japan), Coconade RD, Coconade Sk, Coconade MC, Exepale TGO (Manufactured by Kao Corp., Japan), ODO, TIO (manufactured by the Nisshin Oil Mills, Ltd., Japan), Panasate 800, Psanasate 810, Panasate 1000 (manufactured by Nippon Oil & Fats Co., Ltd., Japan), Trifat S-308 (manufactured by Nikko Chemicals K.K., Japan), Emulex OTG, Emulex KTG (manufactured by Nippon Emulsion K.K., Japan), Captex 300 and 350, (manufactured by Capital City Products Co., U.S.A.), Miglyol 810, 812 and 814 (manufactured by Dynamit Nobel AG, F.R. Germany), Mylitpl 318 (manufactured by Henkel KGaG, F.R. Germany) and Neobee M-5 and O (manufactured by PVO, U.S.A.).

In the present invention, the amount of the triglyceride used as flavor retention ingredient can be suitably determined according to other particular ingredients. Generally, it can be formulated in a dentifrice composition at a level of 0.01 to 5% by weight, preferably 0.1 to 3% by weight based on the total weight of the composition. Particularly, in the present invention, the weight ratio of the triglyceride to flavor contained in the dentifrice composition should be 1 : 9 to 9 : 1 in order to obtain a good flavor retention effect.

Flavor to be used is not specifically limited and any flavor which is generally used for a dentifrice composition can be used in a conventional amount.

Other ingredients are not specifically limited and they may be conventional ingredients. For example, polishing agents such as calcium carbonate, calcium biphosphate, silicic anhydride, aluminum hydroxide, calcium pyrophosphate, insoluble sodium metaphosphate and the like; thickening agents such as sodium carboxymethylcellulose, carrageenan, sodium polyacrylate, sodium alginate, hydroxyethyl cellulose and the like; detergents such as sodium lauryl sulfate, sucrose fatty acid ester, sodium N-lauroylsarcosinate, sodium N-acylglutamate and the like; humectants such as glycerin, sorbitol, polyethylene glycol, propylene glycol and the like; pharmacologically active ingredients such as fluoride, allantoin, aluminum allantoin, chlorhexidine, cetyl pyridinium chloride, $\epsilon$-aminocaproic acid, tranexamic acid, vitamine E and their derivatives, sodium chloride, various enzymes and the like; sweeteners such as saccharin sodium, glycerrhizinate, stevia extract, aspartame, various preservatives can be suitably used in appropriate amounts.

The dentifrice composition of the present invention can be produced according to a conventional method. There is no limitation in the order of addition of the triglyceride and flavor. That is, both can be previously mixed or separately added to give a conventional form such as a toothpaste, gel or a powder dentifrice.

The following experiment, examples and comparative examples further illustrate the present invention in detail but are not to be construed to limit the scope thereof. In following experiment, examples and comparative examples, all %'s are by weight unless otherwise stated.

Experiment

The following samples 1 to 4 were prepared, packed in polyetylene tubes (diameter: 3 cm), sealed in such a way that they appeared like Tetra-packs. Then, the samples were stood at room temperature, and the change of weight with time was measured to determine permeability of dentifrice flavor (peppermint: 35 %; methanol: 25 %; anethole: 5 %; spearment: 5 %; others: 30 %). The results are shown in attached Figure 1. In Figure 1, the ordinate shows decrease in weight (%), and the abscissa shows the passage of time (weeks).

Sample 1: dentifrice flavor 2.5 g + triglyceride (caprylic acid and capric acid (25 : 75)) 2.0 g

Sample 2: dentifrice flavor 2.5 g + glyceride of tricaprylic acid 2.0 g

Sample 3: dentifrice flavor 2.5 g + fat for shortening (hardened fish oil : hardened soybean oil = 90 : 10) 2.0 g

Sample 4: dentifrice flavor 2.5 g

When the triglycerides per se were allowed to stand under the same conditions, they scarcely showed changes in weight thereof. Accordingly, the change in weight of the sample is considered to be caused by the volatilization of the flavor.

As shown in Figure 1, the triglyceride of caprylic acid and capric acid prevents the volatilization of flavor penetrating through the polyethylene tube.

Examples 1 - 3 and Comparative Examples 1 and 2

According to the following formulation, toothpastes containing the triglycerides shown in Table 1 were prepared by a conventional method.

Formulation

| Components | % by weight |
|---|---|
| Silicic anhydride | 15.0 |
| Sodium carboxymethylcellulose | 1.3 |
| Carrageenan | 0.2 |
| Sodium benzoate | 0.1 |
| Sorbitol solution (70% sol.) | 50.0 |
| Concentrated glycerin | 10.0 |
| Polyethylene glycol 400 | 5.0 |
| Sodium lauryl sulfate | 1.3 |
| Saccharin sodium | 0.1 |
| Flavor (peppermint type) | 1.0 |
| Titanium oxide | 0.1 |
| Triglyceride (shown in Table 1) | 0.8 |
| Distilled water | up to 100% |

The resulting toothpastes were packed in plastic tubes (polyethylene) and allowd to stand for one month at 40°C. Volatilization of flavor was estimated by organoleptic test involving six panelists. The results are shown in Table 1. The values in Table 1 mean the number of the panelists who marked the corresponding item.

Table 1

| Triglyceride | Volatilization of Flavor | | | | | Characteristic Odor | | | | |
|---|---|---|---|---|---|---|---|---|---|---|
| | 0 | 1 | 2 | 3 | 4 | 0 | 1 | 2 | 3 | 4 |
| Example 1 (tricaprylic acid glyceride) | 0 | 5 | 1 | 0 | 0 | 6 | 0 | 0 | 0 | 0 |
| Example 2 (triglyceride of caprylic acid and capric acid (25:75)) | 1 | 4 | 1 | 0 | 0 | 6 | 0 | 0 | 0 | 0 |
| Example 3 (triglyceride of mixed fatty acid ($C_6$-$C_{12}$)) | 0 | 4 | 2 | 0 | 0 | 2 | 4 | 0 | 0 | 0 |
| Comp. Example 1 (palm oil (parmitic acid : oleic acid : linolic acid : stearic acid : the others = 42:38:9:4:7)) | 0 | 2 | 3 | 1 | 0 | 0 | 1 | 4 | 1 | 0 |
| Comp. Example 2 (without triglyceride) | 0 | 0 | 0 | 4 | 2 | 6 | 0 | 0 | 0 | 0 |

Note:

The volatilization of flavor and the intensity of odor are expressed according to the following criteria.

0 = no volatilization of odor
1 = scarcely perceptible
2 = distinct
3 = strong
4 = very strong

Example 4
According to the following formulation a toothpaste was prepared by a conventional method.

Formulation

| Components | % by weight |
|---|---|
| Calcium carbonate | 42.0 |
| Silicic anhydride | 1.7 |
| Sodium carboxymethylcellulose | 1.3 |
| Carrageenan | 0.3 |
| Sodium lauryl sulfate | 1.5 |
| Sorbitol solution (70% sol.) | 38.0 |
| Propylene glycol | 4.0 |
| Saccharin sodium | 0.1 |
| Flavor (spearmint type) | 0.9 |
| Triglyceride of caprylic acid and capric acid (1:1) | 0.3 |
| Paraben | 0.2 |
| Distilled water | up to 100% |

The resulting toothpaste was packed in a laminate tube and allowed to stand at 40°C for two weeks but volatilization of flavor scarcely was observed.

Example 5

According to the following formulation, a toothpaste was prepared by a conventional method.

Formulation

| Components | % by weight |
|---|---|
| Aluminum hydroxide | 44.0 |
| Sorbitol solution (70% sol.) | 30.0 |
| Propylene glycol | 2.5 |
| Sodium carboxymethylcellulose | 1.0 |
| Carrageenan | 0.2 |
| Sodium lauryl sulfate | 1.6 |
| Milistic acid diethanol amide | 1.3 |
| Sodium monofluorophosphate | 0.76 |
| Dextranase | 0.02 |
| Paraben | 0.03 |
| Saccharin sodium | 0.1 |
| Flavor (peppermint type) | 1.0 |
| Chlorhexidine hydrochloride | 0.01 |
| Tricaprylic acid glyceride | 1.0 |
| Distilled water | up to 100% |

The resulting toothpaste was packed in a plastic pump dispenser and allowed to stand at 40°C for two weeks but volatilization of flavor was scarcely observed.

Example 6
According to the following formulation, a toothpaste was prepared by a conventional method.

Formulation

| Components | % by weight |
|---|---|
| Silicic anhydride | 23.0 |

| | |
|---|---|
| Sorbitol solution (70 % sol.) | 20.0 |
| Concentrated glycerin | .24.0 |
| Polyethylene glycol 400 | 0.5 |
| Sodium lauryl sulfate | 1.3 |
| Sodium N-lauroylsarcosinate | 0.3 |
| Sodium carboxymethylcellulose | 1.4 |
| Carrageenan | 0.3 |
| Sodium saccharin | 0.1 |
| Paraben | 0.1 |
| Food red No. 106 | Trace |
| Flavor (floralmint type) | 1.0 |
| Triglyceride of mixed fatty acid ($C_6$-$C_{12}$) | 1.5 |
| Distilled water | up to 100% |

The resulting toothpaste was packed in a plastic tube and allowed to stand at 40°C for two weeks but volatilization of flavor was scarcely observed.

Example 7
According to the following formulation, a toothpaste was prepared by a conventional method.

Formulation

| Components | % by weight |
|---|---|
| Calcium phosphate dihydrate for dentifrice | 13.5 |
| Calcium phosphate anhydride for dentifrice | 28.0 |

| | |
|---|---|
| Aluminum oxide | 1.0 |
| Silicic anhydride | 1.7 |
| Sorbitol solution (70% sol.) | 24.0 |
| Propylene glycol | 3.0 |
| Sodium lauryl sulfate | 1.6 |
| Sodium carboxymethylcellulose | 0.2 |
| Sodium benzoate | 0.2 |
| Paraben | 0.4 |
| Chlorhexidine hydrochloride | 0.005 |
| Flavor (doublemint type) | 1.0 |
| Triglyceride of caprylic acid and caproic acid | 0.3 |
| Distilled water | up to 100% |

The resulting toothpaste was packed in a laminate-tube and allowed to stand at 40°C for two weeks but volatilization of flavor was scarcely observed.

**Claims**

1. A dentifrice composition suitable for packing in a container wherein the composition is liable to lose its flavour which comprises as a flavour retaining ingredient a triglyceride of straight or branched chain fatty acids having 6 to 12 carbon atoms, respectively, and other conventional dentifrice ingredients.

2. A dentifrice composition according to claim 1, wherein the weight ratio of the triglyceride to flavour contained in the dentifrice composition is 1:9 to 9:1.

3. A dentifrice composition according to claim 1 or claim 2, wherein the fatty acids contained in the triglyceride are saturated fatty acids having 8 and 10 carbon atoms, respectively.

4. A dentifrice composition according to claim 3, wherein the weight ratio of saturated fatty acid having 8 carbon atoms to that having 10 carbon atoms is 10:1 to 1:8.

5. A dentifrice composition according to any one of the preceding claims wherein the dentifrice composition contains 0.01 to 5% by weight triglyceride.

6. A dentifrice composition according to any one of the preceding claims wherein the dentifrice composition contains 0.1 to 3% by weight triglyceride.

Figure 1